# EUROPEAN PATENT APPLICATION

(11) **EP 2 589 344 A1**
(43) Date of publication of application: **08.05.2013**
(21) Application number: 10854123.6
(22) Date of filing: 12.10.2010
(51) Int. Cl.: A61B 17/02

(54) **MEDICAL DEVICE**

(30) Priority: 30.06.2010 JP 2010149359
(71) Applicant: Terumo Kabushiki Kaisha, Tokyo 151-0072 (JP)
(72) Inventor: TAKAGI Toshiaki, Ashigarakami-gun Kanagawa 259-0151 (JP); ARASE Hideo, Tokyo 100-0005 (JP); OOTANI Yousuke, Ashigarakami-gun Kanagawa 259-0151 (JP); SEKINE Yusuke, Ashigarakami-gun Kanagawa 259-0151 (JP); TAKASHIGE Ryuusuke, Ashigarakami-gun Kanagawa 259-0151 (JP)
(74) Representative: Dossmann, Gérard
(86) International application number: PCT/JP2010/067891
(87) International publication number: WO 2012/001826

(57) **Abstract**

Disclosed is a device capable of providing a space, said space allowing a medical instrument to move, in an area of the brain in which the medical instrument can hardly move. Specifically disclosed is a medical device which comprises a catheter provided with an expandable member, characterized in that said expandable member is expanded to provide a space in which at least a part of a second medical instrument can move.

## Description

### Technical Field

The present invention relates to a medical device for use in an area of the brain, particularly to a medical device making it possible to form a movement-permitting space in which other medical instrument can move.

### Background Art

Conventionally, medical treatment of the brain inside the cranial bones, or so-called brain region treatment, has been carried out through largely exposing the brain through craniotomy by surgical operation. Such an operation exerts a heavy burden on the patient. In addition, in the case where craniotomy is carried out, a long-term hospitalization is needed for recovery. In view of this, in the field of brain surgery, it is desired to perform a medical treatment of a brain region without large-scale craniotomy.

In order to administer a medical treatment substance such as drug solutions, neurotrophic factors, signal transduction substances, gene, cells, etc. to a diseased part present in a brain region, there is conceivable a treatment method in which a catheter is inserted into the brain region.
In the case of an approach via the cranial bones, however, there is no passage and little space in the brain, unlike in a blood vessel where a catheter is often used for medical treatment. Therefore, it is difficult to insert a medical instrument to a site of medical treatment in the brain.
A collection of nerve cells is present at the surface of brain tissue. Therefore, in the case of inserting a medical device while forcing through the brain tissue, there is a risk of damaging the brain tissue with the medical instrument.
In addition, where a medical instrument is inserted while forcing through the brain tissue, the softness of the brain tissue may result in that the position of the medical instrument is instable and it is extremely difficult to achieve the desired treatment.
In the related art, there is found no medical instrument which can be utilized in the case where it is desired to appropriately place a medical instrument in a desired site within the brain region.

Conventionally, in regard of a balloon to be expanded in a blood vessel, there have been known various shapes.
Patent Document 1 describes a balloon which has a double-tube structure and which, when expanded, forms a lumen penetrating therethrough and communicating with the outside of the balloon. In the document, there are described an example in which a catheter is situated in the lumen of the balloon and is adhered to the lumen side of the latter, and a ballooned catheter wherein a catheter is passed through a gap (inside of lumen) between the outer wall and the inner wall of the double-tube structure of the balloon and is adhered in situ.

Patent Document 2 describes expandable double balloon of various shapes as a gastric packing device. A double balloon which is expanded into a hollow cylindrical shape is shown in FIG. 14 of the document.

In the related art, however, there is found no description of a medical device capable of forming a movement-permitting space in which other medical instrument can move.

### Prior Art Documents

### Patent Documents

Patent Document 1: Japanese Patent Laid-open No. Hei 7-532
Patent Document 2: Japanese Patent Laid-open No. 2009-148578

### Summary of Invention

### Technical Problem

It is an object of the present invention to provide a medical device by which a treatment can be carried out safely in a special environment of the brain.

### Technical Solution

The present inventors made earnest investigations for attaining the above object. As a result of their investigations, the present inventors found out that if a space permitting a medical instrument to move therein can be preliminarily formed in a brain region where it is ordinarily difficult to move a medical instrument, the medical instrument can be safely placed in a desired site. Based on the finding, the present invention has been completed.

Accordingly, the present invention provides the following.
(1) A medical device for use in a brain,
   wherein the medical device includes a catheter having an expandable member, and
   the expandable member is expanded to form a space in which at least a part of a second medical instrument can move.
(2) A medical device including: a cylindrical tubular first catheter having a distal end, a proximal end and a first lumen extending therebetween; and an expandable member provided on the first catheter,
   wherein the expandable member, when expanded, can form on the inside thereof a space in which a second medical instrument can move, the first lumen and the movement-permitting space communicate with each other, and at least a part of the second medical instrument can move in the communicating space.

### Advantageous Effects

According to the medical device of the present invention, a required medical instrument can be inserted into a desired site within a brain. In addition, the medical instrument can be placed stably, and a desired treatment can be performed.

### Brief Description of Drawings

[FIG. 1]

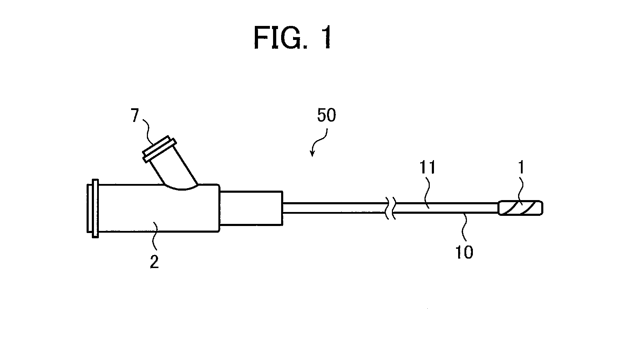

   FIG. 1 is a general view of a medical device according to the present invention.
[FIG. 2]

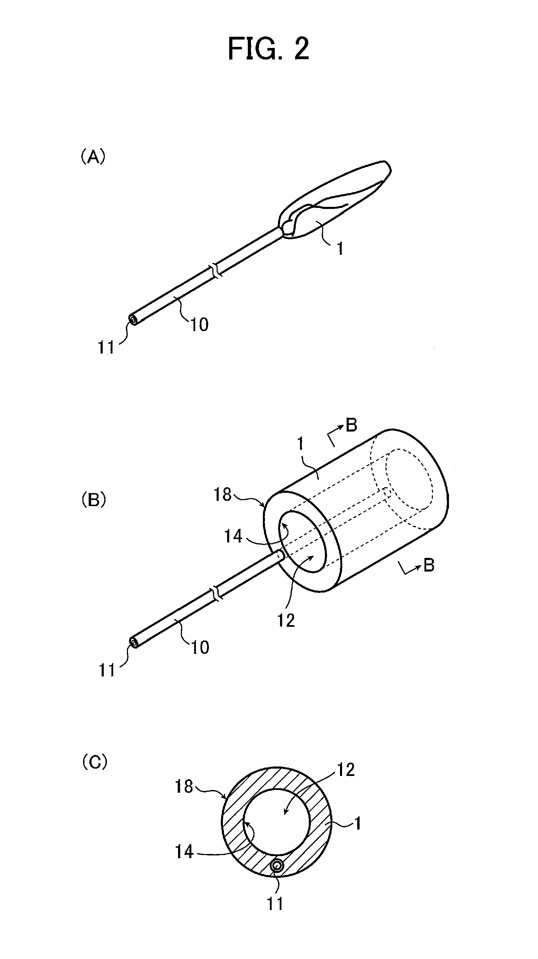

   FIG. 2(A) is a perspective view showing a state in which a balloon 1 before expansion is wrapped around a main body of a first catheter 10. FIG. 2(B) is a perspective view showing a state in which the balloon 1 is expanded into an annular shape. FIG. 2(C) is a sectional view taken along line B-B of FIG. 2(B).
[FIG. 3]

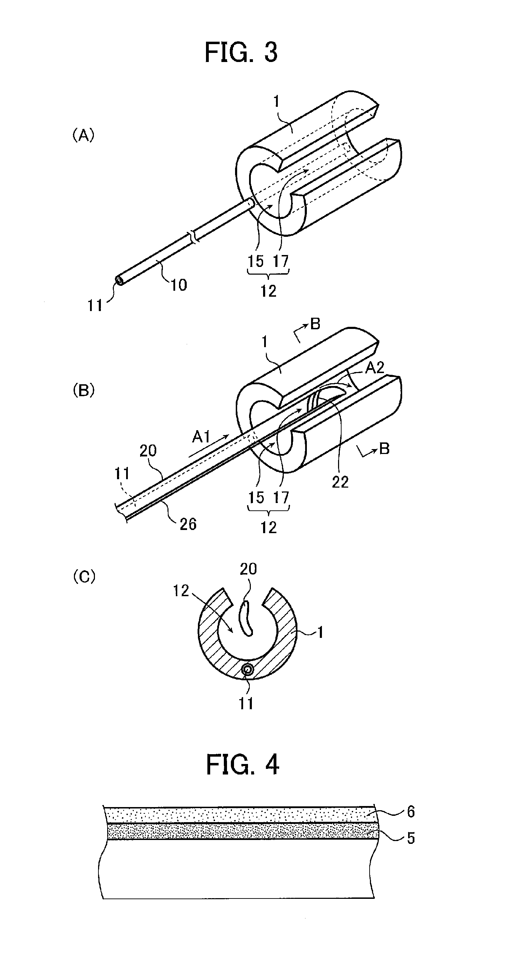

   FIG. 3(A) is a perspective view showing a state in which a balloon 1 is expanded to form a space in which a second medical instrument can move. FIG. 3(B) is a perspective view showing a state in which the second medical instrument 20 is moved in the space 12 thus formed to permit movement therein. FIG. 3(C) is a sectional view taken along line B-B of FIG. 3(B).
[FIG. 4]
   FIG. 4 is a view for illustrating a section of a balloon composed of two layers.
[FIG. 5]

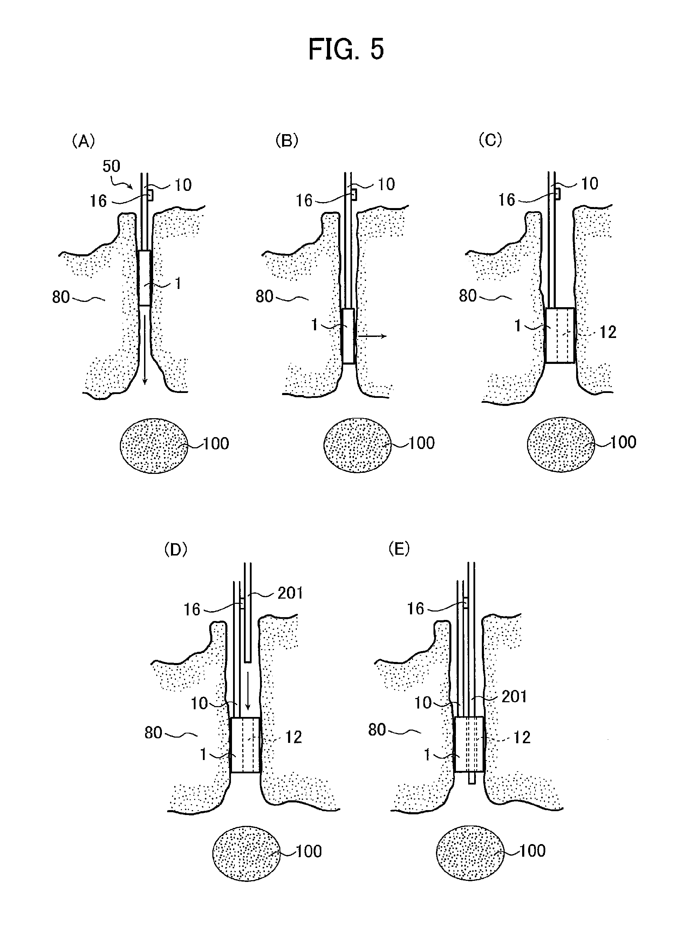

   FIGS. 5(A) to 5(E) are schematic views for illustrating insertion of a first catheter, expansion of a balloon, and movement of a second catheter into a movement-permitting space 12.
[FIG. 6]

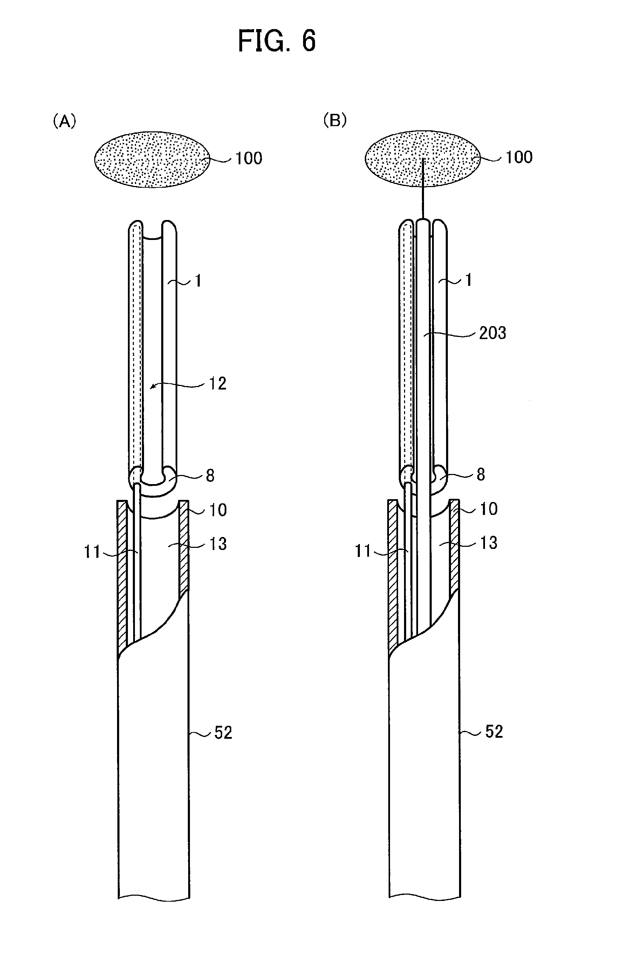

   FIGS. 6(A) and 6(B) are views for illustrating a state in which a movement-permitting space 12 is formed to extend in the major axis direction of a first catheter.
[FIG. 7]

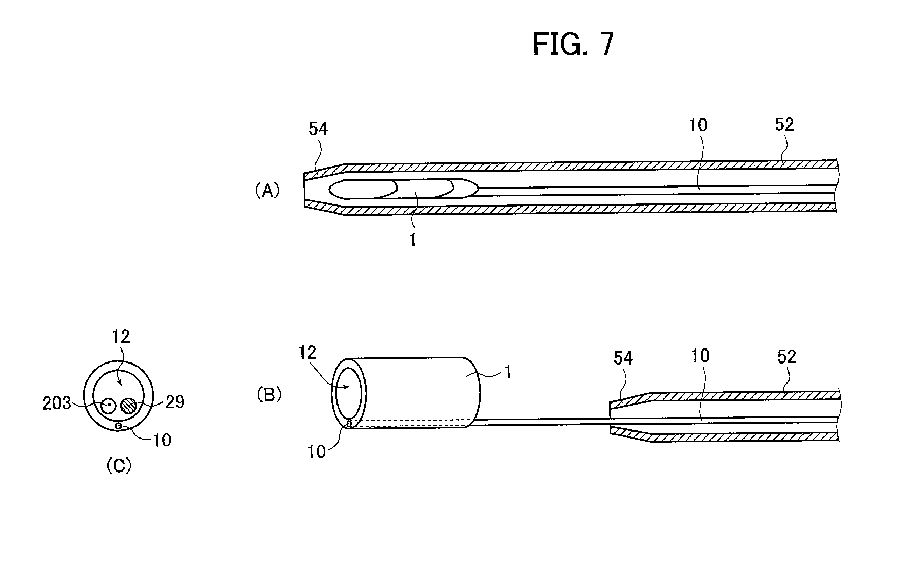

   FIGS. 7(A), 7(B) and 7(C) are views for illustrating steps in Embodiment 1.
[FIG. 8]

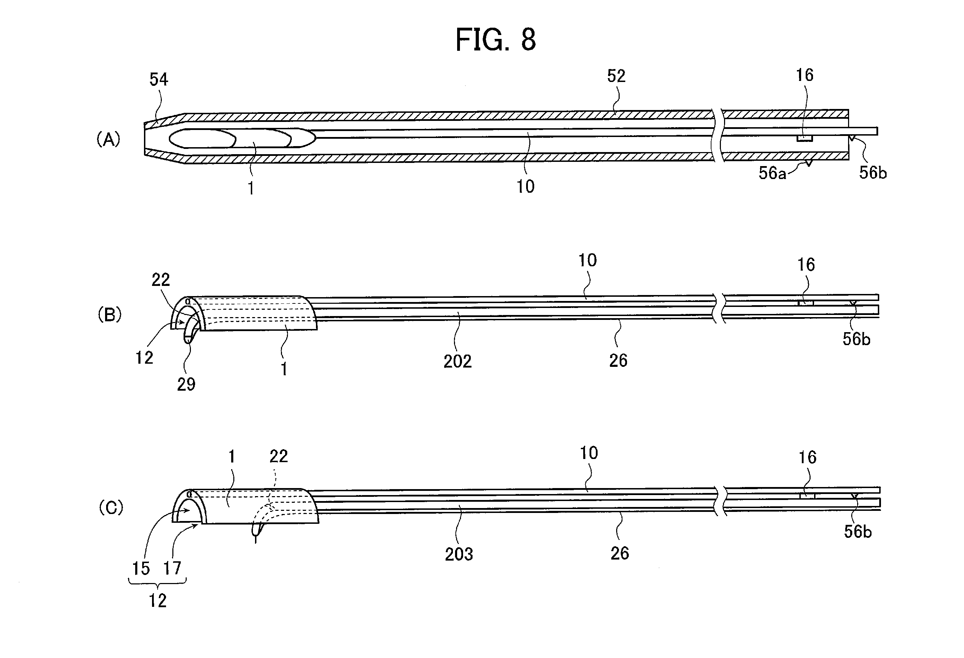

   FIGS. 8(A), 8(B) and 8(C) are views for illustrating steps in Embodiment 2.
[FIG. 9]

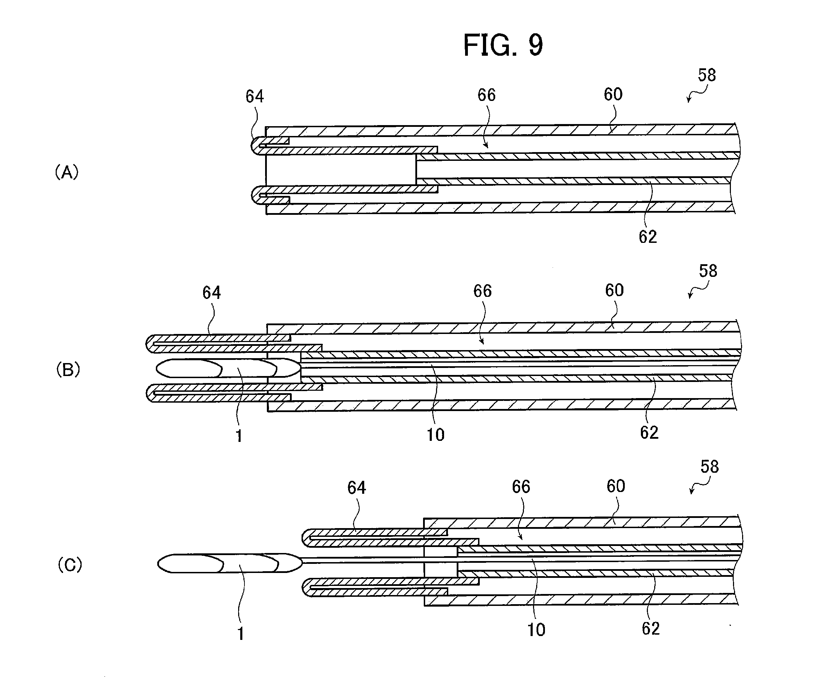

   FIGS. 9(A), 9(B) and 9(C) are views for illustrating part of steps in Embodiment 3.
[FIG. 10]

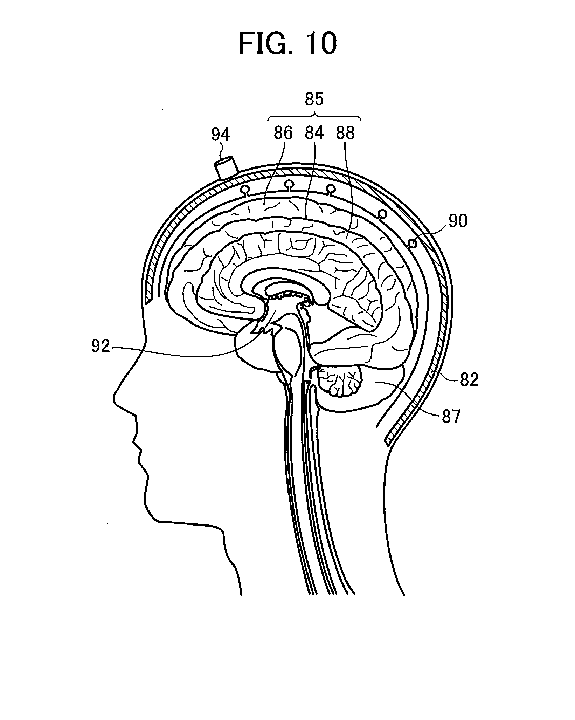

   FIG. 10 is a view for illustrating the inside of the cranial bones.

### Modes for Carrying Out the Invention

Now, a medical device according to the present invention will be described in detail below.
The medical device according to the present invention is a medical device for use in an area of the brain,
wherein the medical device includes a catheter 10 (hereinafter referred to also as first catheter for discrimination from a second medical instrument) having an expandable member 1, and
the expandable member is expanded to form a space in which at least a part of a second medical instrument can move.

FIG. 1 shows an external appearance of the medical device 50 according to the present invention. The medical device 50 includes the first catheter 10, and a branch hub 2 connected to a proximal portion (base end portion) of the first catheter. The catheter 10 is to be inserted to an insertable site in the brain at the time of medical treatment, and has a balloon 1 at the distal end thereof. On the other hand, the branch hub 2 has a fluid port 7 for inflow/outflow of an expansion fluid for expanding the balloon, and the port 7 is in fluid communication with a balloon-expanding lumen 11 in the first catheter. The expansion fluid may be a liquid or a gas. The first catheter 10 may further have one or more lumens other than the balloon-expanding lumen 11.

The expandable member in the present invention is not specifically restricted, and examples of the expandable member include balloons and stents.

Where the expandable member is a balloon, the shape of the balloon is not particularly limited.
Examples of the shape of the balloon after expansion include: a shape which, when expanded, is expanded into a shape being heart-shaped in section and annular in side view, provided on the inside with a cylindrical tubular space and provided at a side surface with a rectangular space formed by cutting out a part of a side surface of an annular shape; a doughnut-like annular shape or rectangular shape not having any cutout (notch); and a shape of a set of two or more members.

FIG. 2(A) is a perspective view showing a state in which a balloon 1 before expansion is wrapped around a main body of a first catheter 10. The first catheter 10 is provided therein with a balloon-expanding lumen 11.
FIG. 2(B) is a perspective view showing a state in which the balloon 1 is expanded into an annular form, by the pressure of a fluid from the balloon-expanding lumen 11, whereby a space 12 in which a second medical instrument can move is formed in a cylindrical tubular shape inside the balloon 1. The first catheter 10 extends through the inside of the side surface of the thus expanded balloon to the distal end of the balloon. The catheter part passed through the inside of the balloon has one or more openings, through which the first catheter 10 communicates with the inside of the balloon and inflow/outflow of an expansion fluid for expanding the balloon can be performed. The catheter 10 may be adhered to the lumen side of the movement-permitting space 10, which is the lumen of the balloon. A configuration may be adopted in which, as shown in FIG. 2(B), the first catheter is inserted between an outer wall 18 and an inner wall 14 of the double-wall tube structure of the balloon and is adhered in situ. Though not shown, a longitudinal member such as a reinforcement wire may be inserted in the lumen of the balloon, particularly along the longitudinal direction, and be adhered in situ.
FIG. 2(C) is a sectional view taken along line B-B of FIG. 2(B).

FIG. 3(A) shows a state wherein a balloon 1 as an expandable member is expanded by the pressure of a fluid from an expanding lumen 11 to form, as a space 12 in which a second medical instrument can move, a shape being heart-shaped in section and annular in side view, provided on the inside with a cylindrical tubular space 15 and provided at a side surface with a rectangular space 17 formed by cutting out a part of a side surface of an annular shape. The first catheter 10 extends through the side surface of the thus expanded balloon to the distal end of the balloon.
FIG. 3(B) shows a state wherein the second medical instrument 20 is moved within the space 12 thus formed to permit movement therein. The second medical instrument 20 is inserted in the direction of arrow A1, next a movable wire 26 firmly attached to a part near the distal end of the second medical instrument 20 is pulled, and a curvable part 22 is curved, whereby the part near the distal end of the second medical instrument 20 is moved in the direction of arrow A2; in other words, the part is moved from the cylindrical tubular space 15 inside the balloon into the rectangular space 17 formed at the side surface of the balloon by cutting out a part of the side surface of the annular shape, as shown in the figure.
FIG. 3(C) is a sectional view taken along line B-B of FIG. 3(B).

The present invention will be described more specifically.
FIGS. 5(A) to 5(E) are schematic views for illustrating the steps of insertion of the medical device according to the present invention into brain tissue 80, expansion of the balloon, movement of a second catheter into the movement-permitting space, and treatment of a part 100 to be treated.
FIG. 5(A) is a view for illustrating a state in which a second catheter 201 as the second medical instrument is guided into the part 100 to be treated by use of the medical device 50 according to the present invention. The direction in which the first catheter 10 of the medical device 50 is inserted is indicated by an arrow. The medical device 50 according to the present invention has a balloon 1 at the distal end of the first catheter 10. Since nerve cells are gathering at the surface of the brain tissue 80, the medical device is inserted while pushing through the brain tissue. During the insertion of the first catheter, the balloon 1 at the distal end is the first to make contact with the brain tissue, so that there is no risk of damaging the brain tissue 80 by insertion of the medical device 50. The fist catheter 10 may be provided at its side surface with a guide 16 or a rail groove by which the second catheter 201 can be guided.
FIG. 5(B) shows an arrow indicative of the direction in which the balloon is expanded.
FIG. 5(C) shows a state upon expansion of the balloon to form a movement-permitting space 12. When the movement-permitting space 12 is formed by expansion of the balloon and the second medical instrument is inserted along the outside of the first catheter, it is possible to secure the space 12 in which the second medical instrument can move with little risk of damaging nerve cells.
FIG. 5(D) is a view for illustrating a state in which the second catheter 201 as the second medical instrument is inserted into the space thus formed to permit movement therein. Up to the distal end of the first catheter, the second catheter 201 can be inserted along the first catheter 10 already inserted.
FIG. 5(E) is a view showing a state wherein the second catheter 201 has been moved within the movement-permitting space 12 and the distal end of the second catheter 201 has arrived at the part 100 to be treated, so that a treatment can be carried out.

The material of the balloon 1 is not particularly limited, and known materials can be used appropriately.
The balloon 1 preferably has a multi-layer structure having a base material layer 5 formed from a high-strength polymer (base layer-forming resin), and a coating layer 6 which is formed on at least a surface of the base material layer 5 and which is formed from a flexible polymer (coating layer-forming resin) more flexible than the high-strength polymer (base layer-forming resin). In the example shown in FIG. 4, the inner layer is the base material layer 5, and the outer layer is the coating layer 6.

The base material layer 5 is formed from a high-strength polymer (base material layer-forming resin). The high-strength polymer (base material layer-forming resin) for use to form the base material layer is preferably a stretchable resin. Examples of the material which can be used include polyethylene terephthalate, polyesters (polyethylene terephthalates) obtained by changing a main acid component or a main glycol component of polyethylene terephthalate, mixtures of these polymers, polyamides (nylon 12, nylon 11, MXD6 nylon), and polyarylene sulfides such as PPS (polyphenylene sulfide).

Examples of the polyesters include those in which isophthalic acid, orthophthalic acid, naphthalenedicarboxylic acid, paraphenylenedicarboxylic acid, cyclohexanedicarboxylic acid, succinic acid, glutaric acid, adipic acid, suberic acid, azelaic acid, sebacic acid, dodecanedioic acid, trimellitic acid, pyromellitic acid, sulfoisophthalic acid, or a salt thereof is used as a main acid component and propylene glycol, butanediol, pentanediol, hexanediol, neopentyl glycol, diethylene glycol, triethylene glycol, polyethylene glycol, polytetramethylene glycol, cyclohexane dimethanol, ethylene oxide adduct of bisphenol A, trimethylolpropane, pentaerythritol or the like is used as a main glycol component.

The material of the coating layer 6 is preferably a flexible polymer (coating layer-forming resin) in the same series as the high-strength polymer (base material layer-forming resin) used for the base material layer, and is further preferably thermoplastic and stretchable. When materials in the same series are used for the base material layer and the coating layer, thermal adhesion or adhesion between both the layers is enhanced. However, a pair of materials enhanced in thermal adhesion or adhesion therebetween by denaturing (modifying) the flexible polymer (coating layer-forming resin) may be used. Besides, a pair of materials which are not in the same series but which are capable of thermal adhesion or adhesion to each other may be used. Furthermore, the base material layer and the coating layer may be provided with an adhesive layer; in this case, the materials may not necessarily be in the same series.

Examples of the flexible polymer (high-polymeric elastomer) for use to form the coating layer include polyester elastomers (for example, those polyester elastomers in which the hard segment is composed of an aromatic polyester and the soft segment is composed of an aliphatic polyether, and those polyester elastomers in which the hard segment is composed of an aromatic polyester and the soft segment is composed of an aliphatic polyester), and polyamide elastomers [for example, those polyamide elastomers in which the hard segment is composed of a polyamide (e.g., nylon 12) and the soft segment is composed of a plasticizer, a polyether or a polyester].

At least a part of that part of the surface coating layer of the balloon 1 which makes contact with the movement-permitting space 12 is preferably surface treated to impart lubricity thereto, which is preferred because smooth movement of the second medical instrument 20 thereon is ensured. Alternatively, an outer surface of the second medical instrument may be surface treated to impart lubricity thereto.
A part of the surface in consideration may be low in lubricity, and examples of a treatment for imparting a low lubricity include such methods as silicone coating and PTFE coating.
Examples of the lubricating treatment include a method of coating with a hydrophilic material. Examples of the hydrophilic material which can be used include maleic anhydride high-polymeric materials (e.g., maleic anhydride copolymers such as methyl vinyl ether-maleic anhydride copolymer). Specific examples of the hydrophilic polymer include high-polymeric compounds having a polymer containing as a constituent a water-soluble monomer (a monomer capable of absorbing the aqueous solvent) such as acrylamide, derivatives thereof, vinylpyrrolidone, acrylic acid and methacrylic acid and their derivatives, etc.
Specific examples of the water-soluble monomer constituting the hydrophilic polymer include N-methylacrylamide, N,N-dimethylacrylamide, acrylamide, acryloylmorpholine, N,N-dimethylaminoethyl acrylate, vinylpyrrolidone, 2-methacryloyloxyethylphosphorylcholine, 2-methacryloyloxyethyl-D-glycoside, 2-methacryloyloxyethyl-D-mannoside, and vinyl methyl ether.
Of the polymer containing the above-mentioned water-soluble monomer as a constituent, the part derived from the water-soluble monomer exhibits surface lubricating properties when wet.
Examples of the lubricating materials which are conceivable include carboxymethyl cellulose, polysaccharides, polyvinyl alcohol, polyethylene oxide, sodium polyacrylate, methyl vinyl ether-maleic anhydride copolymer, and water-soluble nylon.

The first catheter and the second catheter are not particularly restricted, but are formed with a lumen as a straight-formed space section, for inserting a balloon or a wire or the like into a living body. Only one lumen may be formed, or two or more lumens may be formed. The first and second catheters have a desired degree of flexibility. Examples of the material(s) for forming the first and second catheters include polyolefins such as polyethylene, polypropylene, etc., polystyrene, polyamides, polyimides, polyether-ether ketones, polyurethane, polyesters such as polyethylene terephthalate, polybutylene terephthalate, etc., fluoro-resins such as polytetrafluoroethylene, etc., various thermoplastic elastomers based on polyolefin, polystyrene, polyamide, polyurethane, polyester, fluoro-rubber, polyethylene chloride or the like, and, further, combinations (polymer alloys, polymer blends, etc.) of two or more of these materials. The catheters may each have a multi-layer stacked or laminated structure composed of a plurality of materials. In addition, like the above-mentioned balloons, an inner surface or an outer surface of each of the catheters may be treated to impart lubricity to the surface.

Besides, the lengths of the catheters are not particularly limited. For example, the lengths are preferably 5 to 1800 mm, more preferably 5 to 1400 mm.
In addition, the outside diameters of the catheters are not specifically restricted. For instance, the outside diameters are preferably 0.2 to 6.0 mm, more preferably 1.0 to 4.5 mm.
Besides, the inside diameters of the catheters are not particularly limited. For example, the inside diameters are preferably 0.2 to 4.0 mm, more preferably 1.2 to 3.5 mm.

Incidentally, if desired, for preventing the first and second catheters from kinking when bent in the brain and further for enhancing trackability of the catheters with respect to high degrees of bends, the catheters may be provided with a rigidity-imparting body formed in a network or coil form from braided wire or linear (filamentous) body of a high-rigidity material, for example, metals such as stainless steel, elastic metals, superelastic alloys, shape memory alloys, amorphous alloys, etc. or synthetic fibers of polyamide resins, polyester resins, polypropylene resins, etc.

A radiopaque marker may be provided in the vicinity of the distal end, or at a desired position, of each of the catheters. The radiopaque marker is formed from a radiopaque material, and may be provided in a coiled or hollow cylindrical shape. Preferable examples of the radiopaque material include platinum, gold, tungsten, iridium and their alloys, with more preferable examples being platinum, gold, and platinum-iridium alloy. With the radiopaque marker formed from such a material, a clear image of the marker can be obtained under radioscopy, so that the positions of the first and second catheters can be easily confirmed under radioscopy.

FIG. 6(A) shows an example wherein the balloon 1 is expanded while being in the vicinity of the distal end of the first catheter 10. When expanded as shown in FIG. 3, the balloon 1 is expanded while forcing open the brain tissue, to form a space in which the second medical instrument can move. As for the shape of the balloon 1, the balloon 1 is expanded into a shape being heart-shaped in section and annular in side view, provided on the inside with a cylindrical tubular space and provided at a side surface with a rectangular space formed by cutting out a part of a side surface of an annular shape.
FIG. 6(B) shows schematically the manner in which a medical treatment device 203 as the second medical instrument is moved into the movement-permitting space 12, via the lumen inside a guide sheath 52, and a drug solution or the like is inserted into a part 100 to be treated which is present in the vicinity of the distal opening of the balloon 1.

The second medical instrument is not particularly restricted. Examples of the second medical instrument include a shaft equipped with an endoscope, a medical treatment device, a syringe capable of insertion of a drug solution, a knife, an electric cautery, an instrument capable of effecting irradiation with laser light, a wire capable of various operations, an injection needle, capsule of gelled material having a drug solution or cells for medical treatment therein, a patch, a stent, a clamp, a guide wire, and a balloon catheter, of which preferred is the second catheter.

### <Method of guiding the second medical instrument into an area of the brain by use of the medical device according to the present invention>

A method according to the present invention is "a method conducted by use of the above-mentioned medical device according to the present invention, the method including:
inserting the first catheter into a predetermined position in the brain; thereafter expanding the expandable member to form the space 12 in which the second medical instrument can move; and moving at least a part of the second medical instrument in the space, to guide the second medical instrument to the desired position in the brain region." By use of such a method it is possible to guide any of various medical instruments appropriately into a desired site in the brain, in which such insertion is difficult to achieve, safely without damaging brain tissue; it is also possible to stably hold the medical instrument in a desired position, and to carry out various treatments.

A medical device set according to the present invention is a set including the medical device and the second medical instrument according to the present invention which have been described above. The medical device and the second medical instrument according to the present invention may be integrally provided in the same container, for example, tray or packaging bag. This set can be put to manufacture, sterilization, transport, sale, preservation, and use in a collective manner; therefore, the set is convenient for using the medical device of the present invention in a hospital or the like. In addition, the medical device according to the present invention and the second medical instrument for use therewith may be manufactured and sold separately, insofar as they can be recognized to constitute a set. When information on the medical instrument(s) to be combined as a set is attached to the medical device or the second medical instrument, the healthcare staff and patients can purchase and utilize them as a set.

FIG. 10 illustrates the inside of the cranial bones. The brain is protected by being enveloped in a membrane of three layers of connective tissue called meninges. The outermost layer of the meninges, dura, is in close contact with the cranial bones 82. The dura holds the brain in position. The intermediate layer of the meninges is called arachnoid, is composed of fine fibers of connective tissue, and holds the cerebrospinal fluid between itself and the pia mater, which is the innermost layer. In other words, the brain is floating in the cerebrospinal fluid. The brain includes the cerebrum 85 and the cerebellum 87. The cerebrum is divided by the longitudinal fissure of cerebrum into right and left hemispheres, which are the right brain 86 and the left brain 88. At a bottom portion in the skull is a cavity called ventricle, in which many blood vessels are present. The cerebrospinal fluid is secreted from the choroids plexus in the ventricle. The cerebrospinal fluid is circulated in the cavity beneath the arachnoid, before being absorbed into veins through the cerebral venous sinus. The third ventricle 92 is present in a substantially central region.

### <Example 1>

Medical treatment of the third ventricle is conducted by use of the following steps shown in FIGS. 7 and 10.
(1) An opening for insertion of a catheter is formed in the frontal region of head.
(2) The opening is provided with an insertion port 94 shown in FIG. 10. The insertion port is fitted with a valve so that the catheter or the like can be inserted therethrough but the spinal fluid, blood and the like are prevented from flowing out therethrough to the exterior.
(3) The guide sheath 52 shown in FIG. 7(A) is inserted via the insertion port 94. Through the gap in the longitudinal fissure of cerebrum 84, the guide sheath 52 is inserted to a position immediately on the proximal side of the third ventricle, namely, the site of medical treatment. The guide sheath is opening at the distal end thereof so that after insertion of the first catheter, the guide sheath can be moved backward, with the first catheter left in the site into which it has been inserted. The portion near a distal portion 54 of the guide sheath 52 may be tapered (reduced in width) toward the distal end. The distal end is formed of a flexible material, and the edge of an annular shape of the distal end may be rounded so as not to damage the brain tissue.
(4) The first catheter 10 provided at the distal end thereof with a balloon 1 which, when expanded, is doughnut-shaped in section and has a cylindrical tubular space on the inside thereof (hereinafter the expanded balloon will be referred to as annular-shaped balloon) is inserted into the guide sheath 52, and is moved forward to the guide sheath distal portion 54. This state is shown in FIG. 7(A).
(5) The guide sheath 52 is moved backward relative to the balloon before expanded.
(6) Next, as shown in FIG. 7(B), the balloon 1 of the medical device according to the present invention is expanded.
(7) FIG. 7(C) shows a sectional view of the annular-formed balloon thus expanded, specifically showing a state wherein an endoscope 29 and a medical treatment device 203 as the second medical instruments are inserted in the movement-permitting space 12. In practice, the endoscope 29 is inserted along the first catheter 10, and is moved through the movement-permitting space 12 secured by the annular-shaped balloon, to be located at the distal end of the space in the annular-shaped balloon. Then, the part to be treated in the third ventricle (not shown) present on the distal side of the distal end of the annular-shaped balloon is confirmed through the endoscope 29.
(8) Subsequently, the medical treatment device 203 is inserted along the first catheter 10, and is passed through the movement-permitting space 12 secured by the annular-shaped balloon, to be located at the distal end of the space in the annular-shaped balloon. Then, the part to be treated (not shown) located at the distal side of the distal end of the annular-shaped balloon is treated.

### <Example 2>

Medical treatment of a side surface of the longitudinal fissure of cerebrum is carried out by the following steps shown in FIGS. 8 and 10.
(1) An opening through which to insert a catheter is formed in the frontal region of head.
(2) The opening is provided with the insertion port 94 shown in FIG. 10. The insertion port is equipped with a valve so that the catheter can be inserted therethrough but the spinal fluid, blood and the like are prevented from flowing out therethrough to the exterior.
(3) The guide sheath 52 shown in FIG. 8(A) is inserted via the insertion port 94. Through the gap in the longitudinal fissure of cerebrum 84, the guide sheath 52 is inserted into the vicinity of the site of medical treatment.
(4) In the guide sheath 52, the first catheter 10 is inserted. The first catheter thus inserted is moved forward to the distal portion 54 of the guide sheath 52. The first catheter 10 is provided at the distal end thereof with a balloon 1 which, when expanded, is heat-shaped in section and annular in side view, provided on the inside with a cylindrical tubular space and provided at a side surface with a rectangular space formed by cutting out a part of a side surface of an annular shape (hereinafter the expanded balloon will be referred to as heart-shaped balloon). This state is shown in FIG. 8(A).
(5) The guide sheath 52 is moved backward relative to the balloon 1.
(6) As shown in FIG. 8(B), the position of the first catheter 10 is adjusted so that the rectangular space cut out in the side surface of the heart-shaped balloon comes to the site of medical treatment (not shown), and the balloon 1 is expanded to form the movement-permitting space 12. In the example shown in FIG. 8(B), the space formed by expansion of the balloon is substantially semicircular in section. The guide sheath 52 may be provided, at an appropriate position of the outer surface thereof, with a mark 56a for confirmation of the insertion distances of the first catheter 10 and the guide sheath and the direction of cutout in the side surface of the heart-shaped balloon. Alternatively, a mark 56b for position adjustment may be provided at an appropriate position on the first catheter.
(7) An endoscope shaft 202 is moved along the first catheter 10. The endoscope shaft 202 equipped with an endoscope 29 at the distal end thereof and serving as a second medical instrument is passed through the movement-permitting space 12 formed by the heart-shaped balloon. The first catheter 10 may be provided with a guide 16 for guiding the endoscope shaft 202 serving as the second medical instrument. The guide 16 is preferably a structure communicating with the movement-permitting space 12 formed by the balloon. The endoscope shaft 202 is provided with a curvable part 22 in the vicinity of the distal end thereof. A movable wire 26 is fixed to an appropriate position in the vicinity of the distal end of the endoscope shaft 202. The movable wire 26 is passed along the endoscope shaft 202, and can be operated on the side of the proximal end of the first catheter 10. The movable wire 26 is operated and pulled on the side of the proximal end of the endoscope shaft 202, whereby the endoscope shaft 202 is curved at the curvable part 22 thereof, and the endoscope 29 at the distal end thereof is moved from the cylindrical tubular space 15 of the movement-permitting space 12 formed by the heart-shaped balloon 202 into the rectangular space 17 formed in the side surface by cutting out a part of the annular shape (this manner is shown in an enlarged state in FIG. 3(B)). Then, as shown in FIG. 8(B), the site of medical treatment (not shown) at the side surface of the longitudinal fissure of cerebrum 84 is observed and confirmed through the endoscope 29.
(8) Next, a medical treatment device 203 is moved along the first catheter 10, and the medical treatment device 203 as a second medical instrument is passed in the movement-permitting space 12 formed by the heart-shaped balloon. The configuration of the medical treatment device 203 is not specifically restricted; for example, like the endoscope shaft, the medical treatment device 203 may be provided with a curvable part 22 and a movable wire 26. The movable wire 26 is operated and pulled on the side of the proximal end of the medical treatment device 203, whereby the medical treatment device 203 is curved at the curvable part 22 thereof, and the distal end of the medical treatment device 203 is moved from the cylindrical tubular space 15 of the movement-permitting space 12 formed by the heart-shaped balloon into the rectangular space 17 formed in the side surface by cutting out a part of an annular shape. Then, through the opening which is the rectangular space 17 in the side surface of the heart-shaped balloon, a drug solution or the like is administered into the site of medical treatment (not shown) present at the side surface of the longitudinal fissure of cerebrum 84.

### <Example 3>

(1) Step (1) is the same as in Examples 1 and 2.
(2) Step (2) is the same as in Examples 1 and 2.
(3) A collection of nerve cells is present at the surface of brain tissue. In order to insert a medical instrument while forcing through the brain tissue, therefore, it may be preferable to use an everting catheter 58 described in FIGS. 9(A) to 9(C), other than the guide sheath described in Examples 1 and 2. The everting catheter includes an outer tube 60 having a lumen, and an inner tube 62 which can move along the longitudinal direction within the lumen of the outer tube 60 and which has a lumen. The outer tube 60 and the inner tube 62 are in communication with an everting member 64. The everting member 64 is so configured that with the inner tube 62 moved distally within the outer tube lumen, the everting member is everted by passing through the opening of the outer tube 60. The everting member 64 is a thin flexible film formed from an appropriate polymer material. The everting member 64 is joined to the outer tube 60, at a position proximate to the distal opening of the outer tube 60, and to a distal region of the inner tube 62, with an adhesive or by laser fusing, thermal fusing or the like. By actuating an expansion medium in a chamber 66, inversion and eversion of the everting member 64 are operated. By operating the inversion and eversion of the everting member 64, the distal end of the everting catheter 58 is inserted into a site of medical treatment in the brain, while forcing through the brain tissue by the everting member 64. By this operation, the everting catheter 58 can be inserted to a desired position while forcing through the brain tissue, without damaging the nerve cells. Details of the everting catheter are described in Japanese Patent No. 2813463.
(4) The first catheter 10 according to the present invention is passed in the everting catheter 58. The first catheter 10 is moved forward to a distal portion of the everting catheter 58 (see FIG. 9(B)).
(5) The everting catheter 58 is moved backward (see FIG. 9(C)).
The subsequent steps are the same as steps (6) to (8) in Example 1. Alternatively, the same steps as steps (6) to (8) in Example 2 may be carried out.
As has been described above, a collection of nerve cells is present at the surface of the brain tissue. In inserting a medical instrument while forcing through the brain tissue, therefore, the use of the everting catheter described in Example 3 eliminates the risk of damaging the nerve cells. Besides, in the case of inserting a medical instrument while forcing through the brain tissue, the softness of the brain tissue may in some cases result in that the position of the second medical instrument is instable and it is extremely difficult to perform a desired treatment. When a movement-permitting space 12 is formed by use of the first catheter 10 according to the present invention, a medical treatment can be performed in the movement-permitting space 12 formed by the balloon 1 expanded, so that it is possible to carry out the desired treatment, with the position of the second medical instrument made stable.

### Description of Reference Symbols

1 ... Balloon, 2 ... Hub, 5 ... Base material layer, 6 ... Coating layer, 7 ... Port, 8 ... Proximal end, 10 ... First catheter, 11 ... Balloon-expanding lumen, 12 ... Movement-permitting space, 13 ... Lumen, 14 ... Inner wall, 15 ... Space, 16 ... Guide, 17 ... Space, 18 ... Outer wall, 20 ... Second medical instrument, 22 ... Curvable part, 26 ... Wire, 29 ... Endoscope, 50 ... Medical device, 52 ... Guide sheath, 54 ... Distal portion, 56a, 56b ... Mark, 58 ... Everting catheter, 60 ... Outer tube, 62 ... Inner tube, 64 ... Everting member, 66 ... Chamber, 80 ... Brain tissue, 82 ... Cranial bones, 84 ... Longitudinal fissure of cerebrum, 85 ... Cerebrum, 86 ... Right brain, 87 ... Cerebellum, 88 ... Left brain, 90 ... Venous sinus, 92 ... Third ventricle, 94 ... Insertion port, 100 ... Part to be treated, 201 ... Second catheter, 202 ... Endoscope shaft, 203 ... Medical treatment device

## Claims

1. A medical device for use in a brain,
wherein the medical device comprises a catheter having an expandable member, and
the expandable member is expanded to form a space in which at least a part of a second medical instrument can move.

2. The medical device according to claim 1, wherein the space in which at least a part of the second medical instrument can move is formed in a radial direction of the catheter in parallel to a major axis of the catheter.

3. The medical device according to claim 1 or 2, wherein the catheter further has a guide member by which the second medical instrument can be guided.

4. The medical device according to any of claims 1 to 3, wherein the second medical instrument is at least one selected from a second catheter, an endoscope, a medical treatment device and a drug solution infusion tubing.

5. The medical device according to any of claims 1 to 4, wherein the expandable member possessed by the catheter is a balloon, which, when expanded, is expanded into a shape being heart-shaped in section and annular in side view, provided on the inside with a cylindrical tubular space and provided at a side surface with a rectangular space formed by cutting out a part of a side surface of an annular shape.

6. The medical device according to claim 5, wherein the catheter is provided with a mark indicative of the direction of the side surface cutout part.

7. The medical device according to any of claims 1 to 4, wherein the expandable member is a balloon, which, when expanded, forms a cylindrical tubular space being doughnut-shaped in section.

8. The medical device according to any of claims 1 to 7, wherein the catheter further has a guide sheath.

9. The medical device according to claim 8, wherein the guide sheath is an everting catheter.

10. A method of disposing a second medical instrument at a desired position in a brain region, comprising: inserting a medical device comprising a catheter having an expandable member into the brain region; expanding the expandable member after arrival of the expandable member at the desired position, to form on the inside of the expanded member a space in which the second medical instrument can move; and moving at least a part of the second medical instrument in the space thus formed.

11. A medical device set comprising the medical device and the second medical instrument according to any of claims 1 to 9.
